## Europäisches Patentamt

## European Patent Office

## Office européen des brevets

(19)

(11) Veröffentlichungsnummer: **0 060 933**
**B1**

(12)
# EUROPÄISCHE PATENTSCHRIFT

(45) Veröffentlichungstag der Patentschrift:
20.03.85

(51) Int. Cl.⁴: **A 61 K 7/06**, A 61 K 35/34

(21) Anmeldenummer: **81109980.3**

(22) Anmeldetag: **28.11.81**

(54) **Verfahren zum Herstellen eines Haarwuchsmittels.**

(30) Priorität: **12.03.81 DE 3109420**

(43) Veröffentlichungstag der Anmeldung:
**29.09.82 Patentblatt 82/39**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**EP - A - 0 019 301**
**BE - A - 644 474**
**DE - A - 2 004 408**
**FR - A - 972 927**
**FR - M - 2 964**
**GB - A - 990 082**

(73) Patentinhaber: **Kastell, Wolfgang, Milchstrasse 19,
D-2000 Hamburg 13 (DE)**

(72) Erfinder: **Kastell, Wolfgang, Milchstrasse 19,
D-2000 Hamburg 13 (DE)**

(74) Vertreter: **Fleck, Thomas, Dr.Dipl.-Chem. et al,
Patentanwälte Raffay, Fleck & Partner Postfach 32 32 17,
D-2000 Hamburg 13 (DE)**

ACTORUM AG

## Beschreibung

Die vorliegende Erfindung betrifft ein Verfahren zum Herstellen eines Haarwuchsmittels auf Basis von Cytochrome enthaltenden Rinderextrakten.

Ein derartiges Verfahren ist beispielsweise aus der GB-A-990 082 bekanntgeworden, wo angegeben ist, dass Cytochrome zur Kräftigung des Haares verwendbar und nützlich sind. Das Rinderherz wird dort als eine der besten Quellen von Cytochromen beschrieben.

Ferner ist schon aus der EP-A-19 301 ein Haarpflegemittel bekannt, das eine alkohollösliche Fraktion von Sojabohnen-Lecithin in einem niedrigeren Alkohol mit 2 oder 3 Kohlenstoffatomen enthält. Auch werden als Bestandteile in der dortigen Lehre Phosphatidyl-Inosite bzw. Phosphatide, deren Säuren sowie Kephalin angesprochen.

Seit Jahrhunderten wird versucht, durch besondere Zusammensetzungen einfache Mittel herzustellen, die nicht nur den Haarausfall stoppen, sondern auch den Haarwuchs fördern und gleichzeitig die Kopfhaut pflegen. Beim vorliegenden Stand der Technik hat es sich als nachteilig herausgestellt, dass die Herstellungsverfahren für die eingangs genannten Haarwuchsmittel relativ aufwendig sind und häufig nicht einmal die gewünschte Wirkstoffkombination zur Verfügung stellen.

Der vorliegenden Erfindung liegt deshalb die Aufgabe zugrunde, das eingangs genannte Verfahren derart zu verbessern, dass es mit möglichst wenigen Rohmaterialien arbeitet.

Diese Aufgabe wird durch das im Hauptanspruch gekennzeichnete Verfahren gelöst. So werden aus einem einzigen Rohmaterial (Rinderherz) Cytochrome, Phosphatidyl-Inosite, Phosphatide und Phosphatidsäure im geeigneten Mengenverhältnis überraschenderweise gewonnen, denen letztlich nur noch die gewünschte Menge Pflanzenlecithin und evtl. ein alkoholisches Lösungsmittel zugesetzt wird.

Das erfindungsgemässe Verfahren zum Herstellen eines Haarwuchsmittels, wie es im Anspruch 1 durch 17 verschiedene Stufen beschrieben ist, ist im wesentlichen durch die Aufarbeitung eines Rinderherzens gekennzeichnet, bei der auf einmal die gewünschten Wirkstoffe wie Cytochrome, Phosphatidyl-Inosite, Phosphatide und deren Säuren isoliert werden, die dann in den Schritten 16 und 17 mit den Pflanzenlecithinen vereinigt werden.

Den so hergestellten Zubereitungen können ferner die in der Kosmetik üblichen Haar und Kopfhaut pflegenden Substanzen sowie weitere bekannte medizinische Wirkstoffe zugefügt werden. Hingewiesen wird beispielsweise auf Antischuppenmittel, antiseborrhoeische Präparate, sowie Vitamine, Hormone, Konservierungsmittel und weitere ähnliche Stoffe, die selbstverständlich mit den erfindungsgemässen Bestandteilen verträglich sein müssen und den Haarwuchs nicht verhindern dürfen.

Das erfindungsgemäss hergestellte Mittel hat bei zahlreichen Versuchen an Menschen gezeigt, dass nicht nur der Haarausfall verhindert, sondern der Haarwuchs gleichzeitig erheblich geförder wird, wobei die Anagenrate, nämlich der Prozentanteil der in der Wachstumsphase befindlichen Haare, erheblich erhöht wird.

Im folgenden wird die Erfindung anhand eines Ausführungsbeispiels näher erläutert.

*(Herstellungsverfahren):*

250 gr frisches Rinderherz wird mit einem scharfen Messer von den Fettresten befreit und in einem Fleischwolf wie Schabefleisch zerkleinert, so dass ein homogener Brei entsteht, welcher in ein 1 L-Becherglas gegeben wird. Anschliessend wird mit destilliertem Wasser bis zu 0,5 Liter aufgefüllt und mit einem Mixer auf den niedrigsten Stufe der Brei ca. 30 Minuten gerührt.

Danach wird der wässrige Brei mit Schlamm auf einen weichen Faltenfilter gegeben und das wässrige Filtrat aufgefangen. Die Fleischreste werden aufbewahrt.

Das entstandene Filtrat wird kurz aufgekocht, d.h. bis zum Sieden erhitzt. Sobald jedoch ein feiner Niederschlag entsteht, wird sofort abgebrochen und erneut filtriert. Dieser Niederschlag wird jedoch verworfen, während die gewonnene wässrige Lösung als Cytochrom-Lösung bezeichnet werden kann, die bei einer Kühlschranktemperatur von 6°C bis zur Weiterverarbeitung aufbewahrt wird.

Nunmehr werden die nach der ersten Filtration abgetrennten Fleischreste in ca. 250 ml abs. Äther gegeben und in einem luftdicht geschlossenen dunklen Gefäss mit Schraubverschluss zwei Tage bei Raumtemperatur aufbewahrt. Es ist dabei sicherzustellen, dass der Äther nicht entweicht.

Danach wird die entstandene ätherische Lösung filtriert und von den Fleischresten abgetrennt, die dann verworfen werden. Die Lösung wird in einen 1-Liter-Scheidetrichter überführt, wo die wässrige, rotgefärbte von der darüber liegenden ätherischen, gelblich gefärbten Lösung getrennt wird. Die wässrige rot gefärbte Lösung wird verworfen. Bei der Phasentrennung wird der Scheidetrichter zur besseren Phasentrennung eine kurze Zeit lang stehen gelassen. Der Äther wird dann bei Normaldruck unter Verwendung eines elektrisch beheizten Wasserbades abgezogen und der Rückstand anschliessend sofort im Destillationskolben mit 2 mal 75 ml aqua dest. suspendiert. Es wird angenommen, dass diese Suspension im wesentlichen Phosphatidyl-Inosite, freie Phosphatidsäuren und Lecithine enthält.

Diese Suspension wird dann mit der oben aufbewahrten wässrigen Cytochrom-Lösung vereinigt und anschliessend werden 50 g Pflanzenlecithin zugegeben, die aus dem Stärkungsmittel 3N-Lecithin (VEB Arzneimittelwerk Dresden) extrahiert worden sind. Der dann entstandenen Suspension werden 400 ml absoluten Äthanols hinzugegeben. Die Suspension wird mit aqua dest. auf 1 Liter aufgefüllt.

Ferner werden dem so hergestellten Mittel noch Anteile an Ascorbinsäure, insbesondere ca. 1 Gew.%, zugesetzt, um es haltbar zu machen.

Man erhält somit ca. 1 Liter eines alkoholischen Haarwassers in Form einer Suspension, die folgende Hauptbestandteile aufweist:

400 ml abs. Äthanol
400 ml aqua dest.
50 gr Pflanzenlecithine sowie

Cytochrome, Phosphatidyl-Inosite,
freie Phosphatid-Säuren und
Lecithine als wie vorstehend beschrieben hergestellter Extrakt aus 250 g frischem Rinderherz.

**Patentansprüche**

1. Verfahren zum Herstellen eines Haarwuchsmittels auf Basis von Cytochrome enthaltenden Rinderherzextrakten, gekennzeichnet durch folgende Schritte:
   1. Säuberung eines Rinderherzens unter Befreiung von Fettresten;
   2. Zerkleinerung des gesäuberten Herzens, insbesondere Herstellung eines Breies;
   3. Zugabe destillierten Wassers in etwa gleicher Gewichtsmenge des Herzens;
   4. Rühren des Gemisches/Breies oder Stehenlassen des Breies über ca. 24 Stunden im abgeschlossenen Gefäss bei ca. 4°C;
   5. Filtrieren des Gemisches unter Auftrennung in
      a) Filtrat und
      b) Fleischreste;
   6. kurzes Erhitzen des unter 5. gewonnenen Filtrates bis zum Sieden;
   7. nach Entstehung eines feinen Niederschlages erneute Filtration;
   8. ggf. entstandenes Filtrat (Cytochromlösung) bei 4 bis 10° bis zur Weiterverwendung aufbewahren;
   9. Zugabe einer etwa gleichen Gewichtsmenge Äthers zu den unter 5. abgetrennten b) Fleischresten;
   10. Aufbewahrung des unter 9. hergestellten Gemisches für mehrere Stunden bzw. mehrere Tage, insbesondere 2 Tage im geschlossenen Gefäss bei Raumtemperatur;
   11. Filtrieren des unter 10. entstandenen ätherischen Gemisches unter Auftrennung in a) Filtrat und b) Fleischreste;
   12. Abtrennung der ätherischen Phase aus dem unter 11. entstandenen a) Filtrat;
   13. Abdestillieren des Äthers aus der nach 12. entstandenen ätherischen Phase unter Herstellung eines Rückstandes;
   14. Suspendieren des Rückstandes (Phosphatidyl-Inosite, Phosphatide und Phosphatidsäuren) in Wasser;
   15. Vereinigung der unter 14. und 8. hergestellten wässrigen Suspensionen bzw. Lösungen;
   16. Zugabe eines Pflanzenlecithin-Gehaltes, insbesondere etwa 10 bis 15 Gew.-% zu der Suspension nach 15.;
   17. weitere Zugabe von absolutem Äthanol und ggf. Wasser in etwa der gleichen Gewichtsmenge wie die Suspension unter 16.

2. Verfahren nach Anspruch 1, dadurch gekennzeichnet, dass die unter 16. zugegebenen Pflanzenlecithine aus Sojabohnen gewonnen werden.

3. Verfahren nach den vorstehenden Ansprüchen 1 und 2, dadurch gekennzeichnet, dass übliche weiche Papierfilter verwendet werden.

**Claims**

1. A process for preparing a means for the growth of hair on the basis of bovine hearts extracts containing cytochrome, characterized by
   1. cleaning a bovine heart, including removing fatty residues;
   2. mincing the cleaned heart, to form a pasty mixture;
   3. adding distilled water in approximately the same quantity as the heart;
   4. stirring the pasty mixture and allowing it to stand for approximately 24 hours in a sealed vessel at approximately 4°C;
   5. filtering the mixture, and separating it into
      a) a filtrate, and
      b) meat residues;
   6. briefly heating the filtrate obtained in step 5. boiling until a fine precipitate forms;
   7. filtering the fine precipitate and recovering the filtrate;
   8. recovering filtrate, if any (cytochrome-solution) and storing it at 5° - 10°C until further use;
   9. adding approximately the same quantity of ether to the meat residue separated in step 5. b);
   10. storing the mixture prepared in step 9. for several hours or days, respectively, especially 2 days in a sealed vessel at ambient temperature;
   11. filtering the etheral mixture obtained in step 10., and separating into a) filtrate and b) meat residues;
   12. separating the ethereal phase from the filtrate obtained in step 11. a);
   13. distilling the ether from the ethereal phase obtained in step 12., to obtain a residue;
   14. suspending in water the residue comprising phosphatidyl inositols, phosphatides and phosphatic acids;
   15. combining the aqueous suspensions or solutions prepared in steps 8. and 14.;
   16. adding vegetable lecithin, especially in approximately 10 to 15% by weight to the suspension of step 15.;
   17. adding absolute ethanol in approximately the same weight as the suspension of step 16.

2. The process of claim 1, characterized in that the vegetable lecithins added in step 16. are obtained from soya beans.

3. The process according to the preceding claims 1 and 2, characterized in that usual paper filters are used.

**Revendications**

1. Procédé pour la fabrication d'un agent de croissance du cheveu à base d'extraits de coeur de veau contenant des cytochromes, caractérisé par les étapes suivantes:
   1. nettoyage d'un coeur de veau avec élimination des graisses résiduelles;
   2. broyage du coeur nettoyé, en particulier préparation d'une bouillie;
   3. addition d'un poids d'eau distillée à peu près égal au poids du coeur;

4. agitation du mélange/bouillie ou repos de la bouillie pendant environ 24 h dans un récipient fermé à environ 4°C;

5. filtration du mélange avec séparation en
   a) filtrat et
   b) viande résiduelle;

6. court chauffage jusqu'à l'ébullition du filtrat obtenu sous 5.;

7. nouvelle filtration après formation d'un fin précipité;

8. conservation du filtrat éventuellement formé (solution de cytochromes) à 4-10°C jusqu'à l'utilisation ultérieure;

9. addition d'éther à peu près à poids égal à la viande résiduelle b) séparée sous 5.;

10. conservation du mélange préparé sous 9. pendant plusieurs heures ou plusieurs jours, en particulier 2 jours en récipient fermé à la température ambiante;

11. filtration du mélange éthéré formé sous 10. avec séparation en a) filtrat et b) viande résiduelle;

12. séparation de la phase éthérée du filtrat a) formé sous 11.;

13. élimination de l'éther par distillation de la phase éthérée formée sous 12. avec préparation d'un résidu;

14. mise en suspension du résidu (phosphatidylinositols, phosphatides et acides phosphatidiques) dans l'eau;

15. réunion des suspensions ou solutions aqueuses préparées sous 14. et 8.;

16. addition à la suspension selon 15. d'une teneur en lécithine végétale, en particulier, environ 10 à 15% en poids;

17. addition ultérieure d'un poids d'éthanol absolu et éventuellement d'eau à peu près égal au poids de la suspension sous 16.

2. Procédé selon la revendication 1, caractérisé en ce que les lécithines végétales ajoutées sont obtenues à partir du soja.

3. Procédé selon les revendications précédentes 1 et 2, caractérisé en ce que l'on utilise des papiers-filtres souples habituels.